# EUROPEAN PATENT APPLICATION

(11) **EP 3 859 313 A1**
(43) Date of publication of application: **04.08.2021**
(21) Application number: 20154943.3
(22) Date of filing: 31.01.2020
(51) Int. Cl.: G01N 21/64, G01N 21/91, G01N 21/94, G01N 21/954, A61L 2/28

(54) **METHOD AND DEVICE FOR DETECTION OF BIOFILM CONTAMINATION IN ENDOSCOPES**

(71) Applicant: SMP GmbH Prüfen; Validieren; Forschen, 72072 Tübingen (DE)
(72) Inventor: The designation of the inventor has not yet been filed
(74) Representative: Otten, Roth, Dobler & Partner mbB Patentanwälte

(57) **Abstract**

A method for a qualitative and quantitative detection of coatings (4) of biological material, in particular biofilms, on surfaces, in particular in channels and on interior surfaces of tubes (26), preferably on tubular endoscope surfaces, is disclosed. The method comprises: applying a fluorescence dye to the surface to stain the coating, exposing the surface to light to excite a fluorescence emission from the stained coating, and measuring an intensity of the emitted fluorescence light with spatial resolution. The measurement may be performed with a confocal microscope from the outside of the tube if the tube is made of a transparent material, or with an optical probe (40) inserted into the tube, the optical probe comprising an optical fiber and an optical system (42) arranged at an end of the optical fiber and configured to scan a side area of the interior surface of the tube.

## Description

### TECHNICAL FIELD OF THE INVENTION

The invention relates to a method for a qualitative and quantitative detection of coatings of biological material, in particular biofilms, on surfaces and inner channels or tubular surfaces as well as to a device for a corresponding detection. The method can, in particular, be applied to check the contamination level of endoscopes.

### BACKGROUND OF THE INVENTION

Medical instruments as endoscopes are inserted into a part of the human body or an animal for optical detection. Such endoscopes are also used for doing a biopsy. For this purpose, endoscopes have a channel through which parts of the sample that is to be taken can be sucked. After use of an endoscope for medical treatment, e.g. a biopsy, the corresponding parts of the endoscope that came into contact with any biological material inside the body are likely to be contaminated and must be cleaned carefully afterwards. Nevertheless, it must be taken into account that those potentially contaminated surfaces are inside a narrow, long channel of the endoscope, which is hardly accessible, it is difficult to make sure how effective the cleaning process really was. If any biological material as bacteria or other cellular cultures cannot be removed or have survived at hidden places of the inner channel surfaces, they can spread out and form, for example, a biofilm. Such residual biological material is a serious health risk to the next patient that is to be treated. In prior art, such endoscope channels that have already been used and cleaned in a usual manner are checked again on a random basis: The already cleaned endoscope channels are rinsed (for example with purified water) and the liquid, having passed the channel, is analyzed if it contains any biological material. When this is the case, there is evidence for contamination of the channel. Unfortunately this detection method has several disadvantages: First of all, this method only works on condition that any part of the biological material can indeed be detached from the surface during rinsing due to the mechanical forces. If the mechanical forces during rinsing are not enough to detach any part of the coating, the method fails to indicate contamination. Anyhow, the method brings up only few information about potential contamination. On top of that, when any biological material has been found after having rinsed the channel, it is proven that the channel is contaminated, but no other information can usually deduced. Maybe only a few of the actual biomass has been detached, so it is not clear how much residual biological material can still be found in the channel. No quantitative statement or information is possible. Moreover, it cannot be deduced, where the contamination is located inside the channel.

### SUMMARY OF THE INVENTION

The present invention addresses the problem of providing a method to ensure that, using medical instruments, health risks can be avoided in an easier and more reliable manner.

The invention solves the problem using with method according to claim 1 and a device according to claim 11.

By virtue of measures mentioned in the dependent claims, advantageous developments and embodiments of the inventions are possible.

The present invention solves the problem in providing a method to identify contaminated medical instruments, in particular surfaces etc. Typically the method according to the invention can be applied to endoscopes and endoscope channels. Hence the invention presents a method for more qualitative and quantitative detection of coatings of biological material, in particular biofilms, on surfaces, in particular in channels and interior surfaces of tubes, preferably on endoscope surfaces. With the method according to the invention, it becomes possible, for the first time, to do a rapid and quantitative detection of coatings on surfaces of medical instruments, especially in channels or tubes of endoscopes. For this purpose the method according to the invention applies a fluorescence dye to the surface to stain the potential coating on the surface. The dye is supposed to stay only on surfaces that are coated with biological material, in particular biofilm material, where the dye can bind to, intercalate or whose contact provokes an activation of the dye.

The surface is afterwards exposed to light that can excite the fluorescence of the dye. Intensity of the fluorescence light emitted from the stained coating is measured, since it can be supposed that the more coating can be found on the surface, the more dye remains on the surface and contributes to fluorescence.

It is not only possible to qualitatively detect any contamination, but also to get quantitative information about the level of contamination. This part of the analysis can be implemented in two ways: Firstly, measuring the intensity of the emitted fluorescence light, it is possible to get information how much biological material is attached to the surface. Secondly, it is possible to get spatial information where the coating is attached to the surface. In this way using the spatial information, it is possible to clean the surface precisely at the places that are really contaminated and check afterwards in a specific manner, if cleaning was successful.

The quantitative information how much coating can be found on the surface can be used in many different ways: It is possible to deduce from this information how fast the biofilm could have grown on the surface, knowing the latest cleaning or check of the instrument. Furthermore it is possible to get to know how effective the last cleaning process really was. It is possible to see how many cleaning steps or which kind of cleaning process may be necessary to effectively clean the contaminated surface.

The method according to the invention makes sure, in contrast to any other method from prior art, that the surface of a medical instrument is in a suitable condition for being used in any medical treatment without any risk of infecting the patient. The safety of human beings or animals that have to undergo medical treatment can be optimized in that way.

In practice, the dye may attach to surfaces that are not coated with biofilm, cause some background light, remain in the liquid on the surface, even if the sample has been rinsed with purified water after application of the dye. Hence, it can be useful to filter the detected intensity signal with a certain threshold to suppress a small intensity that results from these effects to obtain a more precise result, especially when intensity is used to calculate the biomass.

In the case of an advantageous embodiment, the surface is rinsed or flushed with the fluorescence dye, in particular a solution containing the fluorescence dye, during an exposure time. During that time the dye can attach to, maybe intercalate into the corresponding biological material or be deposited at the coating, depending on the functioning of the dye.

The dye advantageously can penetrate even into a matrix of biological material at the surface. In this way, it can be made sure that approximately the entire biological material gets in contact with the dye, even if the coating consists of a biofilm with a relatively large thickness at some places of the surface. Where the biofilm coating is thicker at certain points on the surface, the dye is in general supposed to need more time to get into the matrix of the biological material and stain also the parts under the surface of the biofilm. In principle, it may be possible to detect the spatial distribution of biological material on the surface, if the dye only stays at the surface of the biofilm, but not the amount of biological material respectively the biomass, if larger amount of the biomass have not been stained.

The more dye can be found on the surface, the higher is the intensity of the emitted fluorescence light, and quantitative detection how much the surface is coated with biological material can be deduced.

In practice, when fluorescence dye is applied to the surface, it is usually not possible to get immediately the clear signal, where the biological material that is to be stained is located, because the dye is also contained in the liquid that surrounds the coating or is, to some extent, deposited on other surfaces, even if there is no coating with biological material. In order to improve the quantitative detection, the surface is flushed with water, in particular demineralized water, to remove the dye that has not bound to biological material or entered the matrix of the biological material. This flushing, rinsing or cleaning step can maybe be done a few times to get a satisfying result and to make sure that the dye is only at the biological material that is to be stained.

For this reason, there is a potential risk that, despite rinsing, the dye has not been removed entirely from the regions where there is no biological material. Advantageously the dye can bind to biological material, but is not deposited on the uncoated surface without the link to the biological material. In many cases the surface is made of PTFE, which prevents the dye in general from binding to an uncoated surface. A suitable dye may be Safranin or crystal violet. Especially Safranin is suitable, since it is not cell-toxic, even if the additional cleaning treatment of the surface cannot remove the entire amount of the fluorescence dye, the remaining amounts of the dye will not harm the person or at least the sample taken from the body that is treated, respectively taken with the medical instrument.

In order to get an optimized quantitative detection signal, the dye can be designed to be activated to fluorescence, only when it is bound to or intercalated into biological material. In this case the binding to the biological material does not only mean that the bound dye is harder to be removed, when the surface is flushed or rinsed. It also means that the probability that a fluorescence signal can be found although the dye is not bound to any biological material, but to an uncoated part of the surface is rather improbable, since the dye at an uncoated part is not activated and cannot contribute to fluorescence. In this way, the signal will only come from those parts of the surface that are coated with biological material.

It was found with an embodiment that, when using a fluorescence dye in an aqueous solution, in particular a 0.03% aqueous solution of crystal violet or 0.05% aqueous solution of Safranin in 1% ethanol may lead to good results. Ethanol can enhance the effect that the fluorescence dye penetrates into the matrix of biofilm. This makes it possible to detect the amount of biological material and biomass in a more precise manner.

Fluorescent light emitted by a molecule is almost always emitted at a longer wavelength than the absorption wavelength. The wavelengths of the excitation (=absorption) light and the detected fluorescence light are defined by the filters within the detector.

Two of the (many potentially compatible) dyes used herein were Safranin-O and Crystal Violet. These dyes have the following spectral properties:
- Safranin-O has an absorption band between 510nm and 530nm, and a fluorescence emission band between 540nm and 590nm.
- Crystal Violet has an absorption band between 500nm and 630nm, and a fluorescence emission band between 610nm and 720nm. The detector used for measurements of these two dyes had the following spectral properties:
- Channel 1 (for Safranin-O) Excitation wavelength 520nm, emission wavelength 570nm
- Channel 2 (for Crystal Violet) Excitation wavelength 625nm, emission wavelength 680nm.
Because of the wavelength separation between excitation and emission (fluorescence) light, the filters in the detector can eliminate excitation light being detected by the emission channel. Even if excitation light is reflected directly back to the detector, this light will not be detected. This minimizes background signals originating from the excitation LED source.

Especially in combination with the detection in narrow channels or tubes, the corresponding optical probe can be small since only one optical fiber or a multi-fiber can be used for detection with an embodiment. In order to improve the spatial resolution, it is possible to use a spatially resolved microscope or confocal microscope. With such a microscope it is possible to detect the coating of the surface even from the outside, for example of the channel, provided that the material the tube is made of is transparent or translucent. The microscope can then be placed outside the tube of the channel and focus the light the dye is exposed to on the area inside the tube on the inside where the coating is expected.

Furthermore, if necessary, it is also possible to detect the thickness of such a coating or to find out where the passage from the coating to the channel material or the tubular material is located. It is advantageous to use confocal microscope optics for such an embodiment.

An embodiment with another advantage uses an optical probe that can also be used as a fluorescence detector at the same time. Such a probe can comprise an optical fiber or a multi-fiber device and can guide the emitted light the dye is exposed to the fluorescence light at the same time. Since the optical fiber is relatively narrow, it is usually possible to insert the probe into a tube or channel over the entire length of the endoscope.

If only the end of the fiber is used to illuminate the sample and couple the fluorescence light back into the fiber end, it is only possible to illuminate and detect lights from a region that is in front of the fiber. With such a system, the spatial resolution is usually relatively imprecise, since a larger area is illuminated and contributes to the detected signal, but it is not possible to get any angular resolution with respect to the longitudinal axis of the tube or any information where the fluorescence light comes from. Therefore an optical system can be used to discriminate between different parts of the surface, if there is any biological coating, for example in form of a biofilm.

With regard to the longitudinal direction of the optical fiber, such optical system can be implemented in different ways:
- In general it is possible to use an optical lens at the end of the fiber to collect the fluorescence light and couple it into the fiber.
- On top of that, it is possible to use a reflecting surface, for example a mirror to project the emitted light to specific areas. The reflecting surface can have a cone shape whose tip is directed towards the optical fiber. It is also possible to use, in combination with the reflecting surface with a cone shape a multi-fiber whose fiber ends are oriented towards the tip of the cone. With this configuration, it is possible to activate not all the fibers at the same time, but different fibers for example in a rotating sense, so that the emitted light is directed to the side area in a rotating manner and the surface can be scanned angularly.

The optical fiber itself can also be covered by a transparent or translucent covering material that surrounds and protects the fiber. The fluorescence light can be coupled into the same fiber again and be detected. It is possible to mount a beam splitter, so that the emitted light has to pass the beam splitter, before it is coupled into the fiber. The beam splitter is also in the optical path of the fluorescence light, so that a part of the light is deviated to another optical path. The fluorescence light can be filtered out by a band-pass filter, since the fluorescence light has usually a longer wavelength than the emitted light, so the fluorescence light is separated.

With an advantages embodiment, it is possible to center the optical probe using a spacer which can be designed as an inflatable balloon to surround the optical probe. The spacer keeps the probe in the center of channel / tube, when it is pushed through the interior of the channel of the tube. On the one hand, the quality of the optical imaging can be improved and furthermore, the optics of the probe is more unlikely to get dirty, because it does not come into contact with the surface and potential biofilms at the surface. The life time of the optical system can be extended.

If the optical probe itself must be cleaned, the spacer can be removed easily and be cleaned separately, so that the sensitive optics can be cleaned with other methods.

### BRIEF DESCRIPTION OF THE DRAWINGS

Exemplary embodiments of the present invention are explained in more detail below, specifying further advantages and further details, and are represented in the drawings.

Specifically, in the drawings:
- Fig. 1-3:: a schematic overview of the steps of the method for detecting biological material on a surface according to the invention;
- Fig. 4:: a schematic view of the inner configuration of an endoscope;
- Fig. 5:: a scheme of an optical set-up to perform the method according to the invention;
- Fig. 6:: a scheme of an optical set-up with a confocal microscope to perform the method according to the invention; and
- Fig. 7:: a scheme of an optical probe to perform the method according to invention.

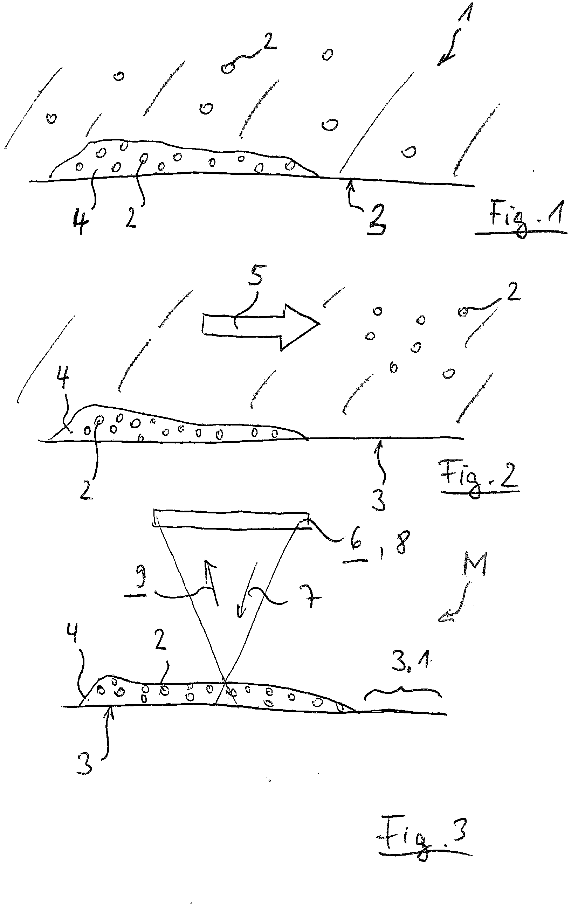

The general method for quantitative and quantitative detection of coatings of biological material on surfaces is shown in Fig. 1 to 3. Figure 1 shows the application 1 of fluorescence dye 2 in an aqueous solution to the surface 3. In some parts the surface 4 is coated with a biofilm 4, which has for example grown on it from residual biological material from the transport of a biological sample over the surface 3 (e.g. during a biopsy).

The dye can penetrate in part into the matrix of the biofilm 4 and binds to the biological material in the biofilm 4. After a certain exposure time the surface 3 is exposed to the dye 2, the sample is flushed with demineralized water to remove those amounts of dye 2 that have not bound to a coating of biological material 4 or entered the matrix of the biofilm 4. The remaining dye 2 which is basically in the aqueous solution is flushed away with the flow 5, as shown in Figure 2. In general the dye 2 cannot bind to the surface 3 in a stable manner, so the uncoated areas 3.1 of the surface 3 remain free from the dye 2.

Figure 3 shows the excitation of a fluorescence by exposing the surface 3 to light of a certain wavelength range. The surface 3 is scanned with a spatially resolved measuring (M) sensor comprising an sensor with an emitter 6, which emits light 7 and exposes the surface 3 to it, and a receiver 8 to detect the fluorescence light 9. The dye 2 is bound to the biofilm 4. Being exposed to light 7 it emits fluorescence light 9. Since the dye can usually not bind to any other part of the surface 3.1, which is not coated with biofilm, the signal resulting from fluorescence comes only from the surface 3 that is contaminated with biofilm 4.

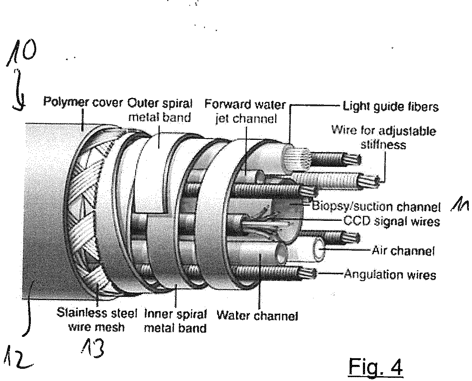

Using the spatially resolved measuring device with emitter 6 and receiver 8, for example a confocal microscope, the detailed places on the surface 3 where the biofilm 4 is located can be detected, as far as the amount of the contamination. A special application of the method is checking endoscopes 10, respectively endoscope tubes / channels 11 for contamination with biofilm material. Figure 4 shows a typical inner configuration of the endoscope 10. The endoscope contains for example a channel 11 in form of a suction channel for biopsy. This biopsy channel 11 is surrounded by other channels for guiding air or water or electrical wires for connecting the lighting devices. The entire assembly is isolated by a polymer cover 12 and screened by a stainless steel wire mesh 13. The critical point is the biopsy channel 11, since biologic material sucked through this channel, so that there is a high risk that parts of the sample remain inside the channel. This biological material may be hard to be removed from the channel, so there is the risk of contamination of the endoscope, because the biological material can also start to grow and form a biofilm that spreads over the inner surface 3.

As already mentioned, although the endoscopes 10 are cleaned after every medical treatment, there is still the risk that some of the biologic material remains inside the very narrow tube.

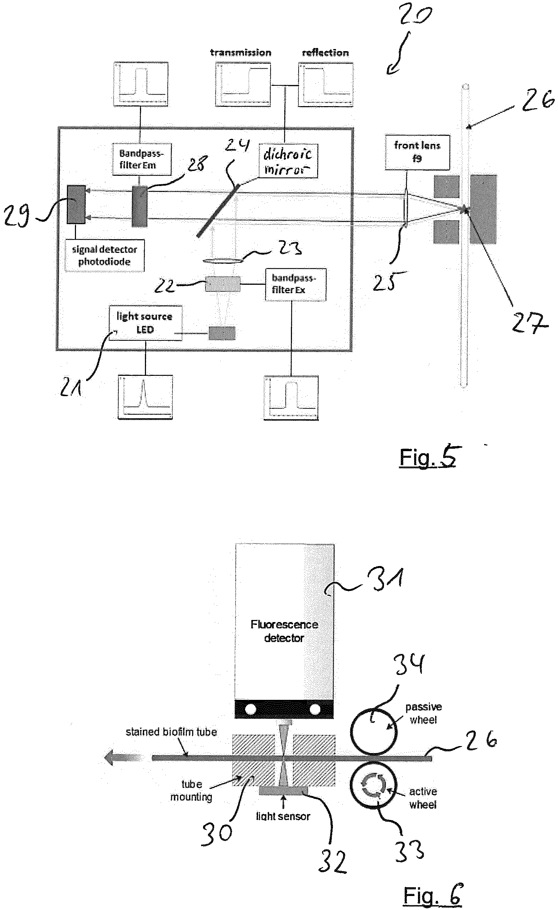

Figure 5 shows a general set-up 20 using a light source 21 to emit the light the surface is exposed to. Depending on the band width of the light source 21, the emitted light can pass a band-pass filter 22 which is adapted to the wavelength for stimulating the fluorescence. In the corresponding optical arrangement 23 the light is sent to a dichroic mirror 24 and guided further via an optical arrangement 25 towards the sample, respectively the tube 26, in particular coupled to an optical fiber and guided towards the sample. In Figure 5 the light is directly focused to the sample, here in form of a PTFE tube 26. If, at position 27, a biofilm has been grown on the surface, this part is stained with the dye and can emit fluorescence light. The fluorescence light is collected by the optical arrangement 25. On its optical path, it passes the dichroic mirror 24 and is filtered by a band-pass filter 28 in order to remove light not emitted by fluorescence with in general smaller wavelength, but the same wavelength as the light emitted from the light source 21. After that, the fluorescence light is detected by the photo diode 29.

Figure 6 shows the tube 26 which is in part stained with a fluorescence dye in the areas where a biofilm has grown. The tube is mounted using a mounting device. The fluorescence detector 31 emits light and detects the fluorescence light. In addition there is a light sensor 32 to detect the state of the light-emitting diode of the fluorescence detector 31. Furthermore in order to scan the sample 26, there is a stepper motor driving the active wheel 33. The passive wheel 34 just stabilizes the tube 26. The motor is switched on every time the light sensor 32 detects light from the fluorescence detector 31. In that manner the tube 26 is scanned with incremental steps.

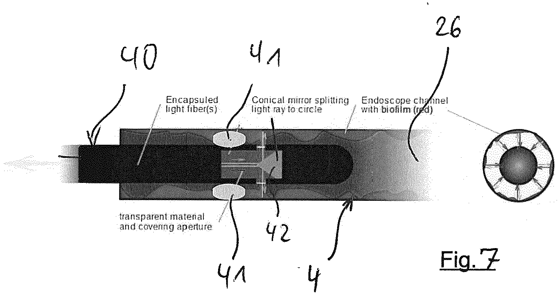

Figure 7 shows the interior of the tube 26 with an optical probe 40 with a set of optical fibers inside. The optical probe 40 has a spacer 41 which surrounds the probe in the certain part to support the probe 40 with respect to the inner surface and center it inside the tube 26. The fibers of the probe 40 emit light that the deviated by a conical mirror 42 to the inner surface of the tube 26. When the surface of the tube 26 is covered with the biofilm, the fluorescence light goes back, is reflected by the mirror 42 and can be coupled again into the fibers. In such a way a controlled angular scanning of the side surface of the tube 26 can be implemented. With a multi-fiber probe it is possible to couple light only to certain fibers and make the emitted light circulate around the longitudinal direction of the endoscope tube 26. In such a way it is possible to scan the interior of the tube.

### LIST OF REFERENCE SYMBOLS

- 1: application of dye
- 2: fluorescence dye
- 3: surface
- 4: biofilm
- 5: flow
- 6: emitter
- 7: emitted light
- 8: receiver
- 9: fluorescence light
- 10: endoscope
- 11: biopsy channel
- 12: polymer cover
- 13: stainless steel mesh
- 20: optical arrangement
- 21: LED source
- 22: band-pass filter
- 23: lens / optics
- 24: dichroic mirror
- 25: optics
- 26: sample / tube
- 27: scanned area
- 28: band-pass filter
- 29: photodiode
- 30: tube mounting
- 31: fluorescence detector
- 32: light sensor
- 33: active wheel
- 34: passive wheel
- 40: optical probe
- 41: spacer
- 42: reflecting mirror (with cone-shape)
- M: spatially resolved measurement

## Claims

1. Method for a qualitative and quantitative detection of coatings (4) of biological material, in particular biofilms, on surfaces (3), in particular in channels (11, 26) and interior surfaces of tubes, preferably on tubular endoscope (10) surfaces, comprising:
• applying (1) a fluorescence dye (2) to the surface (3) to stain the coating (4) on the surface (3),
• exposing the surface to light (7) to excite a fluorescence emission (9) at the stained coating (4),
• spatially resolved measuring (M) of the intensity of the emitted fluorescence light (9).

2. Method according to any of the preceding claims, **characterized in that** applying (1) the fluorescence dye (2) to the surface (3) comprises flushing the surface (3) during an exposure time.

3. Method according to any of the preceding claims, **characterized in that** after having flushed the surface (3), it is flushed (5) with demineralized water.

4. Method according to any of the preceding claims, **characterized in that** a fluorescence dye (2) is used that:
• contains Safranin or crystal violet and/or
• is activated to fluorescence when bound to or intercalated into the biological material.

5. Method according to any of the preceding claims, **characterized in that** a fluorescence dye (2) is used that contains an aqueous solution, in particular a 0.03 % aqueous solution of crystal violet or a 0.05 % aqueous solution of Safranin in 1 % ethanol.

6. Method according to any of the preceding claims, **characterized in that** the surface (3) is exposed to light (7) in the range of 500 nm to 650 nm, in particular 520 nm and/or 625 nm.

7. Method according to any of the preceding claims, **characterized in that** a detector (8, 29) in the range of 500 nm to 700 nm, in particular at 570 nm and/or 680 nm is used.

8. Method according to any of the preceding claims, **characterized in that** the detection is performed using a confocal and spatially resolved microscope (31), whereby, in particular, the microscope is used to detect and/or scan the inner surface (3) of a channel (4) and/or a tube that is made of a translucent material.

9. Method according to any of the preceding claims, **characterized in that** an optical probe (40) is used as fluorescence detector, wherein, in particular, the optical probe comprises an optical fiber and, at end of the fiber, an optical system to scan the side area with regard to longitudinal direction of the optical fiber, and introduced into the channel (4) and/or tube to be scanned.

10. Method according to any of the preceding claims, **characterized in that**
• the amount of biological material of the coating is determined by measuring (M) the intensity of the fluorescence light (9) and/or
• the amount of the covered surface is determined by scanning the surface (3) and detecting where fluorescence light (9) in the expected wavelength range according to the used fluorescence dye (2) is emitted, in particular if intensity of the detected fluorescence light (9) is above a predetermined threshold, and/or
• the amount of biological material of the coating (4) is measured in determining the biomass.

11. Device for a qualitative and quantitative detection of coatings (4) of biological material, in particular biofilms, on surfaces, in particular on the surface (2) of endoscopes (10), preferably on tubular endoscope surfaces, comprising:
• a light source (6, 21) for exposing the surface (3) to light (7) to excite fluorescence emission of stained areas of the surface (3) in a detection area,
• a fluorescence detector (8, 29) for spatially resolved measuring (M) of the intensity of the emitted fluorescence light (9),
• a mounting device (30, 33, 34) with a drive (33) for mounting the surface, in particular the tube (11, 26) of an endoscope (10) and for moving it through the detection area.

12. Device according to any of the preceding claims, **characterized in that** a control device is provided
• to control the position and the already scanned surface area and/or
• to control the penetration depth of the emitted light (7) and the position of the focus in the coating (4) and/or
• to gather the detected light intensities during scanning the surface, in particular assigned to their position on the surface, and/or calculating the integral of the intensities over the spatial area of the surface and/or
• to determine the amount of biological material (4) of the coating, in particular the biomass, by measuring the intensity of the fluorescence light (9),
• to determine the amount of the covered surface (3) by scanning the surface and detecting where fluorescence light (9) in the expected wavelength range according to the used fluorescence dye (2) is emitted, in particular if intensity of the detected fluorescence light (9) is above a predetermined threshold.

13. Device according to any of the preceding claims, **characterized in that** the fluorescence detector (8, 29) comprises an optical probe (40) to be introduced into the channel (11, 26) and/or tube to be scanned, wherein, the optical probe (40) comprises an optical fiber, and, in particular:
• an optical system (42) is arranged at end of the fiber to scan the side area with regard to longitudinal direction of the optical fiber, and/or
• the light source is coupled to the optical fiber to couple the emitted light into the optical fiber and/or
• the fluorescence detector comprises a beam splitter (24) to split off a least a part of the fluorescence light and/or
• a band-pass filter (28) is used to filter out the fluorescence light and/or
• the optical fiber is, at least in part, surrounded by a protective translucent covering material.

14. Device according to any of the preceding claims, **characterized in that** the optical system (29) comprises an optical lens and/or a reflecting surface (42) to project the emitted light to the side area.

15. Device according to any of the preceding claims, **characterized in that** the optical probe (40) comprises a spacer (41), in particular designed as an inflatable balloon to surround the optical probe and center it inside the channel (11, 26) and/or tube to be scanned.
